# EUROPEAN PATENT SPECIFICATION

(11) **EP 4 389 089 B1**
(45) Date of publication and mention of the grant of the patent: **25.11.2026**
(21) Application number: 22215933.7
(22) Date of filing: 22.12.2022
(51) Int. Cl.: A61F 13/02

(54) **A METHOD FOR MANUFACTURING WOUND DRESSINGS**
VERFAHREN ZUR HERSTELLUNG VON WUNDVERBÄNDEN
PROCÉDÉ DE FABRICATION DE PANSEMENTS POUR PLAIES

(43) Date of publication of application: 26.06.2024
(73) Proprietor: Mölnlycke Health Care AB, 431 21 Mölndal (SE)
(72) Inventor: JOHANNISON, Ulf, LANDVETTER (SE); WATHNE, Christian, MÖLNLYCKE (SE)
(74) Representative: Kransell & Wennborg KB

(56) References cited:
- EP-A1- 3 932 374
- KR-A- 20080 084 955
- US-A1- 2020 000 642
- US-A1- 2022 331 167

## Description

### TECHNICAL FIELD

The present disclosure generally relates to a method for manufacturing wound dressings. The method may be performed in a continuous manner and at a high speed and provides wound dressings having an improved flexibility. The present disclosure also relates to a wound dressing formed according to the method.

### BACKGROUND

Adhesive wound dressings are frequently used in wound care, both for the purpose of treating wounds and scars and for the purpose of preventing these from occurring in the first place.

Various characteristics are generally desired when it comes to wound dressings. For example, the wound dressing should be able to handle large amounts of wound exudate. To that end, superabsorbent materials, such as superabsorbent polymer particles or superabsorbent fibers are often included. However, when superabsorbent material is present in the wound dressing, it is important to prevent such material from being in direct contact with the wound of a patient. Leakage of superabsorbent material onto the wound may be detrimental to the wound and the skin of a patient. Skin rashes and skin irritations may result, and the wound healing process may be impaired.

Furthermore, stay-on ability or wear time of the dressing is important. Advanced wound dressings utilized in hospitals and care facilities are typically expensive to produce. Consequently, too frequent dressing changes are associated with high costs.

To improve the wear time of the dressing, the dressing should preferably be flexible such that it can conform to the movement of the patient and remain on the skin when the patient moves.

The flexibility of a wound dressing may be enhanced by providing cuts or incisions in the wound pad of the dressings. However, this may be associated with challenges during manufacturing. This is particularly the case if the wound pad comprises a plurality of layers, and if it comprises a superabsorbent material. A wound pad comprising cuts may enhance the risk of superabsorbent material interfering with the wound or the skin of a patient. Cuts or incisions in the wound pad may also enhance the risk of back-flow of exudate; i.e. wound exudate may flow back towards the wound site during use. A wound dressing comprising cuts is known from EP3932374A1.

Generally, manufacturing of wound dressing involves a chain of steps to assemble a variety of continuous material webs and cutting out individual wound dressings from these web assemblies. Usually, a significant amount of waste material is generated throughout the steps of the process.

There is generally a need to improve the process for manufacturing wound dressings, particularly when involving cutting or providing slits in the wound pads. Such a process should be efficient, economical and be capable of high-speed production. Furthermore, the process should provide for the production of wound dressings having an improved wear time as well as improved liquid handling properties.

### SUMMARY

In view of the above-mentioned problem, it is an object of the present disclosure to provide an improved process for manufacturing wound dressings.

According to a first aspect, there is provided a method for manufacturing wound dressings comprising:
a) providing an absorbent laminate web; the absorbent laminate web comprising at least a first layer and a second layer,
b) providing a plurality of discrete incisions in the absorbent laminate web by feeding the absorbent laminate web into a rotary cutting tool comprising a die cutting roll and an opposing anvil roll, wherein the die cutting roll has an outer surface comprising a plurality of discrete cutting blade structures and wherein the anvil roll has a flat outer surface; the die cutting roll and the anvil roll rotating in opposite directions, wherein the absorbent laminate web is fed into the rotary cutting tool such that the second layer faces the outer surface of the anvil roll and the first layer faces the outer surface of the die cutting roll, and wherein the rotary cutting tool is configured to provide the plurality of discrete incisions in the first layer, but leaving the second layer intact,
c) cutting the absorbent laminate web comprising the plurality of discrete incisions into a plurality of discrete absorbent laminate substrates,
d) arranging the discrete absorbent laminate substrates on a backing layer web such that the first layer of the absorbent laminate substrate faces the backing layer web,
e) applying an adhesive skin contact layer onto the second layer of each of the absorbent laminate substrates, whereby a wound dressing web assembly is provided,
f) cutting out individual wound dressings from the wound dressing web assembly.

The method according to the present disclosure can be performed at a high speed, in a continuous manner, and less waste is generated during the process.

The plurality of discrete incisions in the absorbent laminate web generates no waste material and no material needs to be removed during this process step (step b).

Furthermore, by cutting the absorbent laminate web into a plurality of discrete absorbent laminate substrates (step c) prior to assembly with the backing layer and adhesive skin contact layer results in less material waste compared to if the individual wound dressings would have been cut out after the absorbent laminate web had been assembled with the backing layer and the adhesive skin contact layer.

With the process of the present disclosure, a plurality of discrete incisions are provided in the first layer, which renders the wound pad and the entire dressing more flexible. In this regard, the wear time of the dressing is enhanced since a more flexible dressing that can adapt to the movement of a patient is provided.

The second layer of the absorbent laminate web is the layer configured to be arranged in contact with the adhesive wound contact layer. This layer is void of incisions. This is beneficial since it prevents potential superabsorbent material from reaching the wound and the skin surrounding the wound. In this regard, the second layer acts as a filter layer. Furthermore, the intact second layer prevents back-flow of wound exudate. Exudate back-flow may be particularly prominent when incisions are provided in the absorbent laminate; i.e. wound pad.

The provision of the plurality of discrete incisions after the absorbent laminate has been assembled also saves considerable time compared to the conventional process of providing incisions in one layer and subsequently laminating such a layer with an intact, second layer.

In exemplary embodiments, the discrete cutting blades of the outer surface of the die cutting roll are arranged in close proximity with, but not in direct contact with the outer surface of the anvil roll.

Accordingly, incisions will only be provided in the layer arranged in contact with the outer surface of the die cutting roll.

If the absorbent laminate web comprises a plurality of layers, the method may be tailored to provide incisions in one or several of these layers, but leaving the second layer intact; i.e. void of incisions.

In exemplary embodiments, the first layer of the absorbent laminate web comprises a superabsorbent material, wherein the second layer of the absorbent laminate web is void of superabsorbent material.

The superabsorbent material is preferably not present in close proximity with the wound since the leakage of superabsorbent material may have detrimental effects on the wound and the skin of a patient. Accordingly, in cases where the adhesive skin contact layer is perforated or provided as a coating on the second layer, the risk of leakage of superabsorbent to the wound or skin site is eliminated.

The second layer is not limited to a specific material.

However, in exemplary embodiments, the second layer comprises a foam or a nonwoven.

For example, the second layer may be a polyurethane foam layer or a nonwoven layer.

A polyurethane foam layer has the capacity to absorb and handle large amounts of liquid exuded from the wound site. A nonwoven layer has the ability to spread and distribute wound exudate prior to entry of the wound exudate into the remaining layers of the absorbent laminate; i.e. wound pad. Hence, the liquid handling properties of the final wound dressing is improved.

In exemplary embodiments, the adhesive skin contact layer comprises a polymeric film a provided with an adhesive silicone gel coating.

The adhesive silicone gel coating is configured to contact the skin of a user. The polymeric film yields stability to the adhesive skin contact layer. The adhesive skin contact layer may be provided as a web in the process, and assembled with the remaining components of the wound dressing. The polymeric film yields stability to the adhesive skin contact layer web during the process.

The adhesive silicone gel may be coated onto the polymeric film in advance, and subsequently supplied into the process line as an adhesive skin contact layer web.

Typically, the adhesive skin contact layer comprises a plurality of perforations.

The perforations allow for exudate to enter into the wound dressing such that it can be distributed therein and subsequently be evaporated from the backing layer.

If the adhesive skin contact layer comprises a polymeric film and an adhesive silicone gel coating, the perforations extend through both the polymeric film and the adhesive silicone gel coating.

The adhesive skin contact layer is preferably pre-perforated prior to application onto the absorbent laminate and prior to entry into the process line. This saves additional time during manufacturing.

In step d) of the process, the plurality of discrete absorbent laminate substrates may be arranged onto the backing layer web with a pre-determined distance from one another.

The pre-determined distance sets the size of the border portion that is preferably provided around the contour of each of the absorbent laminate substrates.

The arrangement of the absorbent laminate substrates with a predetermined distance from one another may be accomplished by adjusting the line speed of the backing layer web with the line speed by which the individual absorbent laminate substrates are conveyed onto the backing layer web. The line speed of the backing layer web may be higher than the speed at which the absorbent laminate substrates are applied onto the backing layer web.

The step e) of applying an adhesive skin contact layer may comprise arranging an adhesive skin contact layer web onto the second layer of each of the discrete absorbent laminate substrates, wherein the adhesive skin contact layer web is co-extensive with the backing layer web.

Accordingly, a border portion formed by both the adhesive skin contact layer and the backing layer may be accomplished. This improves the stay-on ability of the final wound dressing.

In step f) of the process, the individual wound dressings may be cut out such that a border portion formed from the backing layer and the adhesive skin contact layer is provided around the contour of each of the discrete absorbent laminate substrates.

Preferably, the method is a continuous automated method.

Accordingly, the method may proceed without interruptions, and no manual steps are required.

In exemplary embodiments, the absorbent laminate web has a lateral (x) extension and a longitudinal (y) extension; the lateral (x) extension corresponding to the machine direction (MD), and wherein the tensile strength of the absorbent laminate web is higher in the lateral (x) direction than in the longitudinal (y) direction.

This is beneficial to provide stability to the process. Furthermore, the step of cutting out absorbent laminate substrates from the absorbent laminate web is significantly simplified. If the absorbent laminate web would be too flexible in all directions in this step, the peripheral edges of the absorbent laminate substrate may break during cutting.

The die cutting roll has an outer surface comprising a plurality of discrete cutting blade structures. The shape of each of the discrete cutting blade structures corresponds to the shape of each discrete incision of the plurality of discrete incisions to be provided in the absorbent laminate web.

In exemplary embodiments, each one of the plurality of discrete cutting blade structures has a shape selected from a cross shape, a star shape, a Y-shape, or an arcuate shape, such as a C-shape.

Accordingly, the plurality of incisions will be provided in the first layer of the absorbent laminate such that this layer is cut with an angle with respect to both the machine direction and the cross-direction. This renders the absorbent laminate substrates, which are to form wound pads in the final wound dressings, more flexible. Hence, the final wound dressing may conform to the movement of a patient, resulting in an enhanced wear time.

In exemplary embodiments, the absorbent laminate web may comprise at least a third layer, wherein the third layer is either arranged on top of the first layer or between the first layer and the second layer, and wherein the third layer is also provided with the plurality of discrete incisions.

The absorbent laminate may comprise various additional layers, which may also be provided with a plurality of discrete incisions to improve the overall flexibility. It is, however, important that the second layer remains intact and void of incisions.

According to another aspect, there is provided a wound dressing manufactured according to the method described hereinbefore, as defined in claim 14.

Further features of, and advantages with, the present disclosure will become apparent when studying the appended claims and the following description. The skilled addressee realizes that different features of the present disclosure may be combined to create embodiments other than those described in the following, without departing from the scope of the present disclosure.

### BRIEF DESCRIPTION OF THE DRAWINGS

The various aspects of the present disclosure, including its particular features and advantages, will be readily understood from the following detailed description and the accompanying drawings, in which:
Figure 1 schematically illustrates a method for manufacturing wound dressings according to an exemplary embodiment of the present disclosure.
Figure 2 schematically illustrates a dressing that may be formed by the method shown in figure 1.

### DETAILED DESCRIPTION

The present disclosure will now be described more fully hereinafter with reference to the accompanying drawings, in which currently preferred embodiments of the present disclosure are shown. The present disclosure may, however, be embodied in many different forms and should not be construed as limited to the embodiments set forth herein; rather, these embodiments are provided for thoroughness and completeness, and fully convey the scope of the present disclosure to the skilled person. Like reference characters refer to like elements throughout.

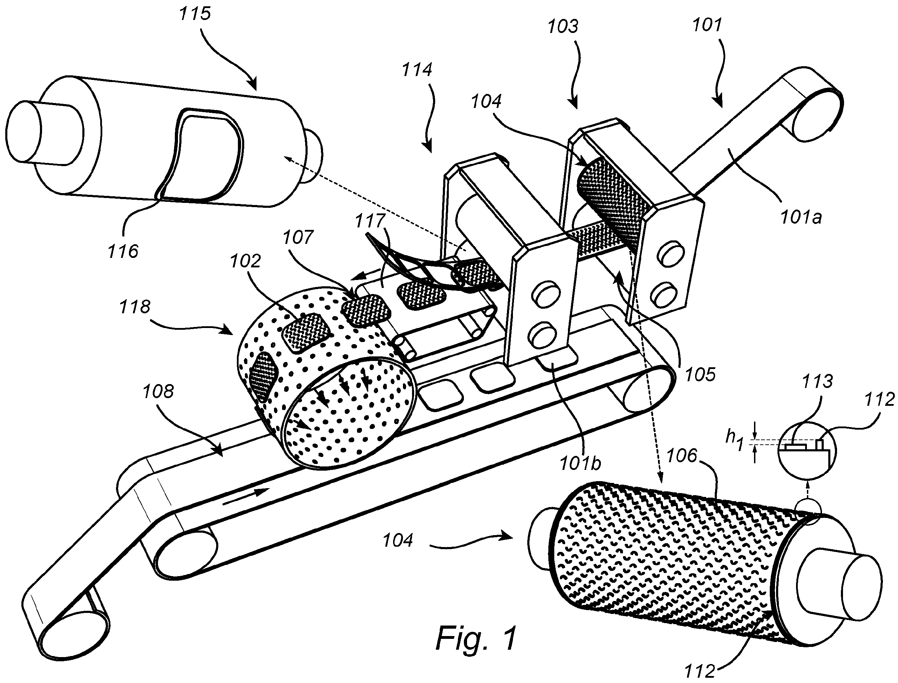

With reference to figure 1, a method for manufacturing wound dressings 100 according to an exemplary embodiment of the present disclosure is conceptually illustrated.

The method comprises:
a) providing an absorbent laminate web 101; the absorbent laminate web 101 comprising at least a first layer 101a and a second layer 101b,
b) providing a plurality of discrete incisions 102 in the absorbent laminate web 101 by feeding the absorbent laminate web 101 into a rotary cutting tool 103 comprising a die cutting roll 104 and an opposing anvil roll 105, wherein the die cutting roll 104 has an outer surface comprising a plurality of discrete cutting blade structures 106 and wherein the anvil roll 105 has a flat outer surface; the die cutting roll 104 and the anvil roll 105 rotating in opposite directions, wherein the absorbent laminate web 101 is fed into the rotary cutting tool 103 such that the second layer 101b faces the outer surface of the anvil roll 105 and the first layer 101a faces the outer surface of the die cutting roll 104, and wherein the rotary cutting tool 103 is configured to provide the plurality of discrete incisions 102 in the first layer 101a, but leaving the second layer 101b intact,
c) cutting the absorbent laminate web 101 comprising the plurality of discrete incisions 102 into a plurality of discrete absorbent laminate substrates 107,
d) arranging the discrete absorbent laminate substrates 107 on a backing layer web 108 such that the first layer 101a of the absorbent laminate substrate 107 faces the backing layer web 108,
e) applying an adhesive skin contact layer 109 onto the second layer 101b of each of the absorbent laminate substrates 107, whereby a wound dressing web assembly is provided,
f) cutting out individual wound dressings from the wound dressing web assembly.

The absorbent laminate web 101 may be provided by means known to the skilled person (step a).

For example, a first laminate layer may be attached to a second laminate layer by means of adhesive, heat, welding or the like. In exemplary embodiments, the first and the second layer of the absorbent laminate web are attached by means of a hot-melt adhesive.

In the context of the present disclosure, the absorbent laminate web 101 is a pre-made absorbent laminate web. Accordingly, the various layers of the absorbent laminate web have been assembled prior to step a) in the process.

As illustrated in figure 1, in step b) of the process, the absorbent laminate web 101 is fed into a rotary cutting tool 103. The rotary cutting tool 103 comprises a die cutting roll 104 and an opposing anvil roll 105.

The die cutting roll 104 and the anvil roll 105 rotate in opposite directions. The outer surface of the die cutting roll 104 faces the outer surface of the anvil roll 105.

The plurality of discrete cutting blade structures 106 provided on the outer surface of the die cutting roll 104 set the shape of the discrete incisions to be provided in the first layer 101a of the absorbent laminate web 101.

As used herein, the term "discrete cutting blade structures" means that the cutting blade structures are arranged separate and isolated from adjacent cutting blade structures. The discrete cutting blade structures are to provide incisions; i.e. slits in the absorbent laminate web. Each cutting blade structure typically has a small width. The width of each cutting blade structure may be between 0.1 and 1 mm.

The distance between adjacent cutting blade structures may be from 1 mm to 15 mm, e.g. from 2 mm to 10 mm. This way, a sufficient area of absorbent laminate material is provided between each discrete incision. Accordingly, the absorbent laminate web may still absorb and handle wound exudate in a proper manner. Furthermore, the risk of rupture or tearing the incised absorbent laminate web is minimized. The provision of incisions (compared to e.g. apertures or channels) also minimizes the risk of exudate flowing back towards the wound site.

The cutting blade structure is not limited to a particular shape. Preferably, each cutting blade structure has a shape which provides incisions provided with an angle to the machine direction (MD) and the cross direction (CD) of the absorbent laminate substrates.

The term "discrete incisions" means that the incisions are arranged separate and isolated from adjacent incisions. The discrete incisions represent slits, wherein as little material as possible is removed during cutting. Preferably, no material is removed.

The shape of each discrete incision corresponds to the shape of each discrete cutting blade structure.

The discrete incisions extend entirely through the first layer. That the second layer is left "intact" means that the discrete incisions do not extend entirely through the second layer.

The discrete cutting blade structures extend radially from the outer surface of the die cutting roll 104. The maximum radial extension, or height, of each cutting blade structure may vary depending on the thickness of the absorbent laminate web, and the thickness of the individual layers comprised therein. The height may be adjusted depending on the characteristics of the second layer of the absorbent laminate web. This layer should be void of incisions.

The cross-sectional area of the die cutting roll 104 in the areas where discrete cutting blade structures 106 are arranged is larger than the cross-sectional area of the die cutting roll 104 in the areas between the cutting blade structures 106.

The anvil roll 105 has a flat outer surface. Accordingly, the anvil roll 105 has an even outer surface. In the areas of the anvil roll 105 where the absorbent laminate web is to be conveyed, the cross-sectional area of the anvil roll is the same. There are consequently no variations in diameter across the anvil roll 105.

The second layer 101b faces and is arranged to contact the outer surface of the anvil roll 105. The first layer 101a faces and is arranged to contact the outer surface of the die cutting roll 104.

The rotary cutting tool 103 is configured to provide the plurality of discrete incisions 102 in the first layer 101a, but leaving the second layer 101b intact.

To achieve this, the discrete cutting blades 106 may be arranged in close proximity with, but not in direct contact with the outer surface of the anvil roll 105.

The distance between the outermost edges 113 of the cutting blade structures 106 and the outer surface of the anvil roll 104 may be adjusted depending on the thickness of the second layer 101b of the absorbent laminate web 101.

The outermost edges of the cutting blade structures define the maximum radial extension; i.e. height, of the cutting blade structures.

The distance between the outermost edges of the cutting blade structures and the outer surface of the anvil roll may be from 0.02 to 1.0 mm, e.g. from 0.03 to 0.8 mm.

The distance may be regulated by any means known to the skilled person.

In exemplary embodiments, and as illustrated in figure 1, the distance between the cutting blade structures 106 and the outer surface of the anvil roll 105 may be regulated by the provision of raised side sections 112 on the die cutting roll 104. The diameter of the side sections 112 is thus larger than the maximum diameter of the die cutting roll 104 in the areas where the discrete blade structures 106 are arranged. Accordingly, there will be a distance in height, h1, between the side sections 112 and the outermost edges 113 of the cutting blade structures 106. The height, h1, may be regulated depending on the thickness of the second layer 101b of the absorbent laminate web 101.

It is also conceivable that the distance between the outermost edges of the cutting blade structures and the outer surface of the anvil roll is regulated in a different manner. For example, the anvil roll may be an adjustable anvil roll. The anvil roll may e.g. comprise three separate sections, such as a first side section, a second side section and a third central section arranged between the first and the second side sections. The third, central section may be movable and lowered with respect to the side sections. Hence, the height, h1, may be adjusted in this, alternative manner.

Step c) of cutting the absorbent laminate web 101 comprising the plurality of discrete incisions 102 into a plurality of discrete absorbent laminate substrates 107 is not limited to a specific method or technique.

As illustrated in figure 1, step c) may be performed by feeding the absorbent laminate web 101 comprising the plurality of discrete incisions 102 into a contour cutting tool 114.

The contour cutting tool 114 is configured to cut through all the layers of the absorbent laminate web 101.

The contour cutting tool 114 may comprise a roll 115 having an outer surface comprising a cutting blade profile 116 corresponding to the shape of the absorbent laminate substrate 107 to be formed.

The shape of the absorbent laminate substrates 107 is in figure 1, illustrated as square shaped, however the absorbent laminate substrates are not limited to such a shape. For example, the shape of the absorbent laminate substrates 107 may be rectangular, oval, circular or any other conceivable shape. The shape of the absorbent laminate substrate 107 may be regulated by the contour cutting tool 114.

The step d) of arranging the discrete laminate substrates 107 on a backing layer web 108 such that the first layer 101a of the absorbent laminate substrate 107 faces the backing layer web 108 may be performed by any suitable technique known to the skilled person.

For example, as illustrated in figure 1, after the step of cutting the absorbent laminate web 101 by the contour cutting tool 114, the discrete absorbent laminate substrates 107 may be arranged on a conveyor belt 117. Subsequently, the absorbent laminate substrates 107 may be arranged on a conveyor roll 118. Vacuum (illustrated by the arrows in figure 1) may be applied to secure that the individual absorbent laminate substrates 107 remain on the surface of the conveyor roll 118.

The absorbent laminate substrates are arranged on the backing layer web 108 such that the first layer 101a of the absorbent laminate substrate 107 faces the backing layer web 108. The first layer 101a may be arranged in contact with the backing layer web 108, as illustrated in figure 1.

The present disclosure is by no means limited to the specific conveyor belts and rolls illustrated in figure 1, but any means for conveying the various webs and materials is conceivable.

The step e) of applying an adhesive skin contact layer onto the second layer 101b of the absorbent laminate substrates 107 may be provided by means known to the skilled person.

For example, the adhesive skin contact layer may be provided as a coating directly on the absorbent laminate substrates 107 and/or the backing layer web 108.

Alternatively, the adhesive skin contact layer is provided as an adhesive skin contact layer web and applied to the second layer 101b of the absorbent laminate 101.

The step f) of cutting out individual wound dressings from the wound dressing web may be performed by any means known to the skilled person.

The various layers of the wound dressings may be attached to one another by conventional means, such as by means of adhesives.

The first layer 101a of the absorbent laminate web 101 may comprise a superabsorbent material, wherein the second layer 101b of the absorbent laminate web 101 is preferably void of superabsorbent material.

As used herein, the term "superabsorbent material" means a material comprising superabsorbent polymers (SAP). A superabsorbent polymer can absorb more than 100 times its own weight in aqueous fluids. Superabsorbent polymers are constituted by water-swellable and water insoluble polymers capable of absorbing large quantities of fluid upon formation of a hydrogel. The superabsorbent polymers for use in accordance with the present disclosure may be inorganic or organic crosslinked hydrophilic polymers, such as polyvinyl alcohols, polyethylene oxides, crosslinked polyacrylates and the like. Typically, the superabsorbent (SAP) comprise sodium acrylate. The SAP material may be in the form of particles, fibers, flakes, powder or similar. Preferably, the SAP material is in the form of superabsorbent polymer (SAP) particles or fibers (SAF).

The second layer 101b of the absorbent laminate web 101 is a continuous layer, void of incisions and superabsorbent material.

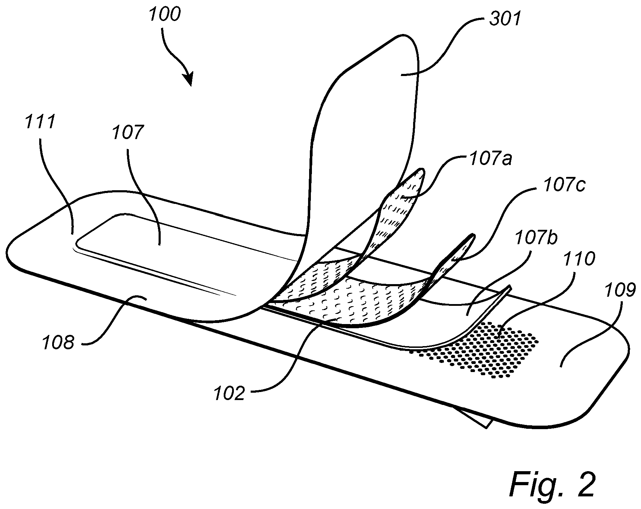

The second layer 101b of the absorbent laminate web 101 represents the layer 107b of the absorbent laminate substrate 107 to be arranged in contact with the adhesive skin contact layer 109 in the wound dressing 100 formed by the method of the present disclosure. (see figure 2).

In figure 2, the various absorbent laminate layers of the absorbent laminate substrate 107 are denoted 107a-c. The first layer 107a of the absorbent laminate substrate 107 is formed from the first layer 101a of the absorbent laminate web 101. The second layer 107b of the absorbent laminate substrate 107 is formed from the second layer 101b of the absorbent laminate web 101. The dressing of figure 2 also comprises a third layer 107c arranged between the first 107a and the second 107b layer of the absorbent laminate substrate 107. The third layer 107c may be formed from a third layer arranged between the first 101a and the second 101b layers of the absorbent laminate web 101.

Accordingly, the features and characteristics discussed in relation to the layers 101a-c of the absorbent laminate web 101 apply to the absorbent laminate substrate layers 107a-c.

The lack of superabsorbent material in the second layer 101b eliminates the risk of SAP leakage to the wound site and the skin during use of the final wound dressing. Such leakage could potentially occur if the adhesive skin contact layer is perforated.

The second layer 101b may comprise a foam or a nonwoven.

The foam is preferably hydrophilic and absorbent. The absorbent foam has the ability to efficiently absorb wound exudate and transport such exudate away from the wound site.

Preferably, the foam is a hydrophilic and porous polyurethane foam. The polyurethane foam may comprise pores having an average pore size in the range of from 30 and 1000 µm.

The second layer 101b may, alternatively, be a nonwoven layer. A nonwoven layer has the ability to distribute fluid efficiently such that the wound exudate is spread over a larger surface area. This is beneficial both when exudate is transported from the wound site, and also when exudate is transported backwards towards the wound site.

The nonwoven layer may comprise a meltblown, spunbond or a spunlaced nonwoven. Examples of suitable polymers for use in the nonwoven are polyethylene, polyesters, polypropylene and other polyolefin homopolymers and copolymers. For example, nonwoven webs comprising thermoplastic fibers of polypropylene and polyethylene fibres or mixtures thereof may be used. The webs may have a high content of thermoplastic fibres and contain at least 50%, e.g. at least 70% thermoplastic fibres. The nonwoven may be a mixture of polyester and viscose, e.g. in a 70:30 ratio. The basis weight of the nonwoven may be in the range of from 10 to 80 g/m2, e.g. of from 20 to 50 g/m2. The liquid spreading layer may also be a spunbond- meltblown or spunbond-meltblown-spunbond (SMS) web.

The adhesive skin contact layer 109 may comprise a polymeric film provided with an adhesive silicone gel coating.

The polymeric film yields stability to the adhesive skin contact layer 109. The adhesive skin contact layer is preferably pre-fabricated such that the adhesive skin contact layer can be supplied in the process and applied to the absorbent laminate substrates 107 as an adhesive skin contact layer web.

The polymeric film is preferably a breathable film. The polymeric film may comprise polyethylene, polyamide or polyurethane. Preferably, the polymeric film comprises polyurethane. The thickness of the polymeric film may be from 15 to 100 µm, e.g. from 20 to 80 µm, preferably from 20 to 50 µm.

Examples of suitable silicone gels for use in the adhesive silicone gel coating include the two component RTV systems, such as Q72218 (Dow Corning), and SilGel 612 (Wacker Chemie AG) mentioned herein, as well as NuSil silicone elastomers. In embodiments of the invention the adhesive may comprise a soft silicone gel having a softness (penetration) of from 8 to 22 mm, e.g. from 12 to 17 mm, as measured by a method based on ASTM D 937 and DIN 51580, the method being described in European Patent Application No 14194054.4. The thickness of the silicone gel layer is typically at least 20 µm. The thickness of the silicone gel layer may be from 100 to 200 µm.

Preferably, the adhesive skin contact layer 109 comprises a plurality of perforations 110.

The perforations extend through the entire adhesive skin contact layer; i.e. through both the polymeric film and the adhesive silicone gel coating, if present.

As illustrated in figure 2, the perforations may be provided at least in the area of the adhesive skin contact layer 109 underlying the absorbent laminate substrate 107; i.e. the wound pad. In the embodiment disclosed in figure 2, the area of the adhesive skin contact layer 109 forming part of the border portion 111 is void of perforations.

Alternatively, the perforations are provided across the entire surface of the adhesive skin contact layer 109.

As illustrated in figure 1, in step d) the plurality of discrete absorbent laminate substrates 107 may be arranged onto the backing layer web 108 with a pre-determined distance from one another.

The pre-determined distance may vary depending on the size of the border portion of the wound dressing. For example, the pre-determined distance may be from 5 to 60 mm, e.g. from 10 to 40 mm. Accordingly, a sufficiently sized border portion is accomplished.

The arrangement of the absorbent laminate substrates with a predetermined distance from one another may be regulated by the line speed of the backing layer web 108; i.e. the speed at which the backing layer web 108 is conveyed. The line speed of the backing layer web 108 may be higher than the speed at which the absorbent laminate substrates 107 are conveyed. In figure 1, the speed of the absorbent laminate substrates 107 may be regulated by the conveyor belt 117, but the process is not limited thereto. The line speed of the absorbent laminate web 101 may, in embodiments, be lower than the speed of the backing layer web 108.

The step e) of applying an adhesive skin contact layer 109 may comprise arranging an adhesive skin contact layer web onto the second layer 101b of each of the discrete absorbent laminate substrates 107, wherein the adhesive skin contact layer web is co-extensive with the backing layer web 108 (not shown in figure 1).

The adhesive skin contact layer web and the backing layer web 108 may be co-extensive in length and width. The adhesive skin contact layer web and the backing layer web 108 may have the same surface area.

In step f), the individual wound dressings may be cut out such that a border portion formed from the backing layer and the adhesive skin contact layer 109 is provided around the contour of each of the discrete absorbent laminate substrates 107.

The backing layer of the final wound dressing will thus have the same width and length as the width and length of the adhesive skin contact layer.

Accordingly, as illustrated in figure 2, both the backing layer 108 and the adhesive skin contact layer 109 form part of the border portion 111 of the final wound dressing.

The border portion 111 improves the adhesion to the skin of a patient.

Preferably, the method is a continuous automated method.

Accordingly, the method may be carried out at a high speed and without any interruptions or manual steps.

The absorbent laminate web 101 may have a lateral (x) extension and a longitudinal (y) extension; the lateral (x) extension corresponding to the machine direction (MD), and wherein the tensile strength of the absorbent laminate web 101 is higher in the lateral (x) direction than in the longitudinal (y) direction.

This is beneficial from a process stability point of view. Furthermore, when the absorbent laminate web 101 enters the contour cutting tool 114, the peripheral edges of the absorbent laminate substrates 107 are prevented from breaking or tearing apart during cutting.

The discrete cutting blade structures 106 is not limited to a specific shape as long as they are configured to provide incisions; i.e. not apertures or larger holes, in the absorbent laminate web.

Preferably, each one of the plurality of discrete cutting blade structures 106 of the die cutting roll 104 has a shape selected from a cross shape, a star shape, a Y-shape, or an arcuate shape, such as a C-shape.

These shapes provide incisions in the absorbent laminate web, which enhances the overall flexibility of the final wound dressings.

In figure 1, C-shaped cutting blade structures 106 are illustrated.

The absorbent laminate web 101 may comprise at least a third layer, wherein the third layer is either arranged on top of the first layer 101a or between the first layer 101a and the second layer 101b, and wherein the third layer is also provided with the plurality of discrete incisions 102.

The third layer of the absorbent laminate web may be a liquid transport layer, a liquid distribution layer and/or a liquid absorption layer.

In figure 2, a wound dressing comprising an absorbent laminate substrate 107 with three laminate layers (107a-c) is illustrated.

The wound dressing 100 comprises a backing layer 108, an adhesive skin contact layer 109 and an absorbent laminate substrate 107 comprising at least a first layer 107a and a second layer 107b, wherein the first layer 107a comprises a superabsorbent material and being provided with a plurality of discrete incisions 102 and wherein the second layer 107b is arranged in contact with the adhesive skin contact layer 109 and being void of superabsorbent material and discrete incisions 102.

In figure 2, a third layer, denoted 107c is arranged between the first 107a and the second 107b layers. The third layer 107c is provided with a plurality of discrete incisions 102. The flexibility of the absorbent laminate substrate 107 and the dressing is thereby improved.

The second layer 107b is arranged to contact the adhesive skin contact layer 109. This layer is intact, which is beneficial to prevent back-flow of exudate, and to prevent superabsorbent material from leaking to the wound site.

The dressing 100 may comprise additional layers and is by no means limited to the construction illustrated in figure 2.

In figure 2, the adhesive skin contact layer 109 comprises a plurality of perforations 110 in the area underlying the absorbent pad, but is void of apertures in the area forming the border portion 111.

The adhesive skin contact layer 109 is arranged to receive body fluids, e.g. wound exudate from the wound while the absorbent laminate substrate 107 serves as a wound pad and is configured to absorb the wound exudate and transport it away from the wound by evaporating it from the top of the dressing; i.e. through the backing layer 108.

With reference to the dressing illustrated in figure 2, the first layer 107a be a layer comprising a superabsorbent material, preferably superabsorbent fibers (SAF), the second layer 107b may be an absorbent foam layer, and the third layer may be a may be a nonwoven layer,.

Alternatively, the first layer 107a may be a tissue layer, the second layer 107b may be a nonwoven layer, and the third layer 107c may be a layer comprising a superabsorbent material. It is also conceivable that absorbent laminate comprises two layers. Hence, the first layer 107a may be a layer comprising superabsorbent particles or fibers, and the second layer 107b may be a nonwoven layer.

Terms, definitions and embodiments of all aspects of the present disclosure apply mutatis mutandis to the other aspects of the present disclosure.

Even though the present disclosure has been described with reference to specific exemplifying embodiments thereof, many different alterations, modifications and the like will become apparent for those skilled in the art.

Variations to the disclosed embodiments can be understood and effected by the skilled addressee in practicing the present disclosure, from a study of the drawings, the disclosure, and the appended claims. Furthermore, in the claims, the word "comprising" does not exclude other elements or steps, and the indefinite article "a" or "an" does not exclude a plurality.

## Claims

1. A method for manufacturing wound dressings comprising:
a) providing an absorbent laminate web (101); said absorbent laminate web (101) comprising at least a first layer (101a) and a second layer (101b),
b) providing a plurality of discrete incisions (102) in said absorbent laminate web (101) by feeding said absorbent laminate web (101) into a rotary cutting tool (103) comprising a die cutting roll (104) and an opposing anvil roll (105), wherein said die cutting roll (104) has an outer surface comprising a plurality of discrete cutting blade structures (106) and wherein said anvil roll (105) has a flat outer surface; said die cutting roll (104) and said anvil roll (105) rotating in opposite directions, wherein said absorbent laminate web (101) is fed into said rotary cutting tool (103) such that said second layer (101b) faces the outer surface of said anvil roll (105) and said first layer (101a) faces the outer surface of said die cutting roll (104), and wherein said rotary cutting tool (103) is configured to provide said plurality of discrete incisions (102) in said first layer (101a), but leaving said second layer (101b) intact,
c) cutting said absorbent laminate web (101) comprising said plurality of discrete incisions (102) into a plurality of discrete absorbent laminate substrates (107),
d) arranging said discrete absorbent laminate substrates (107) on a backing layer web (108) such that said first layer (101a) of said absorbent laminate substrate (107) faces said backing layer web (108),
e) applying an adhesive skin contact layer (109) onto said second layer (101b) of each of said absorbent laminate substrates (107), whereby a wound dressing web assembly is provided,
f) cutting out individual wound dressings from said wound dressing web assembly.

2. The method according to claim 1, wherein said discrete cutting blades (106) of said outer surface of said die cutting roll (104) are arranged in close proximity with, but not in direct contact with said outer surface of said anvil roll (105).

3. The method according to claim 1 or claim 2, wherein said first layer (101a) of said absorbent laminate web (101) comprises a superabsorbent material and wherein said second layer (101b) of said absorbent laminate web (101) is void of superabsorbent material.

4. The method according to any one of the preceding claims, wherein said second layer (101b) comprises a foam or a nonwoven.

5. The method according to any one of the preceding claims, wherein said adhesive skin contact layer (109) comprises a polymeric film provided with an adhesive silicone gel coating.

6. The method according to any one of the preceding claims, wherein said adhesive skin contact layer (109) comprises a plurality of perforations (110).

7. The method according to any one of the preceding claims, wherein in said step d) said plurality of discrete absorbent laminate substrates (107) are arranged onto said backing layer web (108) with a pre-determined distance from one another.

8. The method according to any one of the preceding claims, wherein said step e) of applying an adhesive skin contact layer (109) comprises arranging an adhesive skin contact layer web onto said second layer (101b) of each of said discrete absorbent laminate substrates (107), wherein said adhesive skin contact layer web is co-extensive with said backing layer web (108).

9. The method according to any one of the preceding claims, wherein in said step f), said individual wound dressings are cut out such that a border portion (111) formed from said backing layer and said adhesive skin contact layer (109) is provided around the contour of each of said discrete absorbent laminate substrates (107).

10. The method according to any one of the preceding claims, wherein said method is a continuous automated method.

11. The method according to any one of the preceding claims, wherein said absorbent laminate web (101) has a lateral (x) extension and a longitudinal (y) extension; said lateral extension (x) corresponding to the machine direction (MD), and wherein the tensile strength of said absorbent laminate web (101) is higher in the lateral (x) direction than in the longitudinal (y) direction.

12. The method according to any one of the preceding claims, wherein each one of said plurality of discrete cutting blade structures (106) of said die cutting roll (104) has a shape selected from a cross shape, a star shape, a Y-shape, or an arcuate shape, such as a C-shape.

13. The method according to any one of the preceding claims, wherein said absorbent laminate web (101) comprises at least a third layer (101c), wherein said third layer (101c) is either arranged on top of said first layer (101a) or between said first layer (101a) and said second layer (101b), and wherein said third layer (101c) is also provided with said plurality of discrete incisions (102).

14. A wound dressing manufactured according to the method of any one of claims 1-13, wherein said wound dressing comprises a backing layer (108), an adhesive skin contact layer (109) and an absorbent laminate substrate (107) comprising at least a first layer (107a), a second layer (107b), and a third layer (107c) arranged between said first (107a) and second (107b) layers
wherein said first layer (107a) comprises a superabsorbent material and being provided with a plurality of discrete incisions (102) and wherein said second layer (107b) is arranged in contact with said adhesive skin contact layer (109) and being void of superabsorbent material and discrete incisions (102), wherein said third layer (107c) is provided with a plurality of discrete incisions (102).

## Patentansprüche

1. Verfahren zum Herstellen von Wundverbänden, umfassend:
a) Bereitstellen einer absorbierenden Schichtstoffbahn (101); wobei die absorbierende Schichtstoffbahn (101) mindestens eine erst Schicht (101a) und eine zweite Schicht (101b) umfasst,
b) Bereitstellen einer Vielzahl von getrennten Einschnitten (102) in der absorbierenden Schichtstoffbahn (101) durch Zuführen der absorbierenden Schichtstoffbahn (101) in ein rotierendes Schneidwerkzeug (103), das eine Stanzwalze (104) und eine gegenüberliegende Gegendruckwalze (105) umfasst, wobei die Stanzwalze (104) eine Außenfläche aufweist, die eine Vielzahl von getrennten Schneidklingenstrukturen (106) umfasst, und wobei die Gegendruckwalze (105) eine ebene Außenfläche aufweist; wobei sich die Stanzwalze (104) und die Gegendruckwalze (105) in entgegengesetzte Richtungen drehen, wobei die absorbierende Schichtstoffbahn (101) dem rotierenden Schneidwerkzeug (103) derart zugeführt wird, dass die zweite Schicht (101b) der Außenfläche der Gegendruckwalze (105) zugewandt ist und die erste Schicht (101a) der Außenfläche der Stanzwalze (104) zugewandt ist, und wobei das rotierende Schneidwerkzeug (103) so ausgelegt ist, dass es die Vielzahl von getrennten Einschnitten (102) in der ersten Schicht (101a) bereitstellt, die zweite Schicht (101b) jedoch intakt lässt,
c) Zuschneiden der absorbierenden Schichtstoffbahn (101), die die Vielzahl von getrennten Einschnitten (102) umfasst, zu einer Vielzahl von getrennten absorbierenden Schichtstoffsubstraten (107),
d) Anordnen der getrennten absorbierenden Schichtstoffsubstrate (107) auf einer Trägerschichtbahn (108) derart, dass die erste Schicht (101a) des absorbierenden Schichtstoffsubstrats (107) der Trägerschichtbahn (108) zugewandt ist,
e) Aufbringen einer haftenden Hautkontaktschicht (109) auf der zweiten Schicht (101b) von jedem der absorbierenden Schichtstoffsubstrate (107), wodurch eine Wundverbandbahn-Anordnung bereitgestellt wird,
f) Herausschneiden von einzelnen Wundverbänden aus der Wundverbandbahn-Anordnung.

2. Verfahren nach Anspruch 1, wobei die getrennten Schneidklingen (106) der Außenfläche der Stanzwalze (104) nahe bei der Außenfläche der Gegendruckwalze (105) angeordnet sind, jedoch nicht in unmittelbarem Kontakt damit.

3. Verfahren nach Anspruch 1 oder Anspruch 2, wobei die erste Schicht (101a) der absorbierenden Schichtstoffbahn (101) ein superabsorbierendes Material umfasst und wobei die zweite Schicht (101b) der absorbierenden Schichtstoffbahn (101) kein superabsorbierendes Material umfasst.

4. Verfahren nach einem der vorhergehenden Ansprüche, wobei die zweite Schicht (101b) einen Schaum oder ein Vlies umfasst.

5. Verfahren nach einem der vorhergehenden Ansprüche, wobei die haftende Hautkontaktschicht (109) eine Polymerfolie umfasst, die mit einer haftenden Silikongelbeschichtung versehen ist.

6. Verfahren nach einem der vorhergehenden Ansprüche, wobei die haftende Hautkontaktschicht (109) eine Vielzahl von Löchern (110) umfasst.

7. Verfahren nach einem der vorhergehenden Ansprüche, wobei die Vielzahl von getrennten absorbierenden Schichtstoffsubstraten (107) in Schritt d) mit einem vorher festgelegten Abstand zueinander auf der Trägerschichtbahn (108) angeordnet werden.

8. Verfahren nach einem der vorhergehenden Ansprüche, wobei Schritt e) zum Aufbringen einer haftenden Hautkontaktschicht (109) Anordnen einer Bahn für eine haftende Hautkontaktschicht auf der zweiten Schicht (101b) von jedem der getrennten absorbierenden Schichtstoffsubstrate (107) umfasst, wobei die Bahn für eine haftende Hautkontaktschicht dieselbe Erstreckung aufweist wie die Trägerschichtbahn (108).

9. Verfahren nach einem der vorhergehenden Ansprüche, wobei in Schritt f) die einzelnen Wundverbände derart herausgeschnitten werden, dass ein Randabschnitt (111), der aus der Trägerschicht und der haftenden Hautkontaktschicht (109) gebildet ist, um den Umriss von jedem von den getrennten absorbierenden Schichtstoffsubstraten (107) herum bereitgestellt wird.

10. Verfahren nach einem der vorhergehenden Ansprüche, wobei das Verfahren ein kontinuierliches automatisiertes Verfahren ist.

11. Verfahren nach einem der vorhergehenden Ansprüche, wobei die absorbierende Schichtstoffbahn (101) eine Querausdehnung (x) und eine Längsausdehnung (y) aufweist; wobei die Querausdehnung (x) der Maschinenrichtung (MD) entspricht und wobei die Zugfestigkeit der absorbierenden Schichtstoffbahn (101) in der Querrichtung (x) höher ist als in der Längsrichtung (y).

12. Verfahren nach einem der vorhergehenden Ansprüche, wobei jede von der Vielzahl von getrennten Schneidklingenstrukturen (106) der Stanzwalze (104) eine Form aufweist, die aus der Form eines Kreuzes, der Form eines Sterns, der Form eines Y oder einer gebogenen Form wie der Form eines C ausgewählt ist.

13. Verfahren nach einem der vorhergehenden Ansprüche, wobei die absorbierende Schichtstoffbahn (101) mindestens eine dritte Schicht (101c) umfasst, wobei die dritte Schicht (101c) entweder oben auf der ersten Schicht (101a) oder zwischen der ersten Schicht (101a) und der zweiten Schicht (101b) angeordnet wird und wobei die dritte Schicht (101c) ebenfalls mit der Vielzahl von getrennten Einschnitten (102) versehen ist.

14. Wundverband, der nach dem Verfahren nach einem der Ansprüche 1-13 hergestellt ist, wobei der Wundverband eine Trägerschicht (108), eine haftende Hautkontaktschicht (109) und ein absorbierendes Schichtstoffsubstrat (107) umfasst, das mindestens eine erste Schicht (107a), eine zweite Schicht (107b) und eine dritte Schicht (107c) umfasst, die zwischen der ersten (107a) und der zweiten Schicht (107b) angeordnet ist, wobei die erste Schicht (107a) ein superabsorbierendes Material umfasst und mit einer Vielzahl von getrennten Einschnitten (102) versehen ist, und wobei die zweite Schicht (107b) im Kontakt mit der haftenden Hautkontaktschicht (109) angeordnet ist und kein superabsorbierendes Material und keine getrennten Einschnitte (102) umfasst, wobei die dritte Schicht (107c) mit einer Vielzahl von getrennten Einschnitten (102) versehen ist.

## Revendications

1. Procédé de fabrication de pansements pour plaies comprenant :
a) la prévision d'une bande stratifiée absorbante (101) ; ladite bande stratifiée absorbante (101) comprenant au moins une première couche (101a) et une deuxième couche (101b),
b) la réalisation d'une pluralité d'incisions discrètes (102) dans ladite bande stratifiée absorbante (101) en apportant ladite bande stratifiée absorbante (101) dans un outil de coupe rotatif (103) comprenant un rouleau de coupe à l'emporte-pièce (104) et un rouleau d'enclume opposé (105), ledit rouleau de coupe à l'emportepièce (104) ayant une surface extérieure comprenant une pluralité de structures de lames de coupe discrètes (106) et ledit rouleau d'enclume (105) ayant une surface extérieure plate ; ledit rouleau de coupe à l'emporte-pièce (104) et ledit rouleau d'enclume (105) tournant dans des directions opposées, ladite bande stratifiée absorbante (101) étant introduite dans ledit outil de découpe rotatif (103) de telle sorte que ladite deuxième couche (101b) fasse face à la surface extérieure dudit rouleau d'enclume (105) et que ladite première couche (101a) fasse face à la surface extérieure dudit rouleau de coupe à l'emporte-pièce (104), et ledit outil de coupe rotatif (103) étant configuré pour créer ladite pluralité d'incisions discrètes (102) dans ladite première couche (101a), mais en laissant ladite deuxième couche (101b) intacte,
c) la découpe de ladite bande stratifiée absorbante (101) comprenant ladite pluralité d'incisions discrètes (102) en une pluralité de substrats stratifiés absorbants discrets (107),
d) la disposition desdits substrats stratifiés absorbants discrets (107) sur une bande de couche support (108) de telle sorte que ladite première couche (101a) dudit substrat stratifié absorbant (107) fasse face à ladite bande de couche support (108),
e) l'application d'une couche adhésive de contact avec la peau (109) sur ladite deuxième couche (101b) de chacun desdits substrats stratifiés absorbants (107), ce qui permet d'obtenir un ensemble de bande de pansement,
f) la découpe des pansements individuels dans ledit ensemble de bande de pansement.

2. Procédé selon la revendication 1, dans lequel lesdites lames de coupe discrètes (106) de ladite surface extérieure dudit rouleau de coupe à l'emporte-pièce (104) sont agencées à proximité immédiate de ladite surface extérieure dudit rouleau d'enclume (105), mais non en contact direct avec celle-ci.

3. Procédé selon la revendication 1 ou la revendication 2, dans lequel ladite première couche (101a) dudit voile stratifié absorbant (101) comprend un matériau super-absorbant et dans lequel ladite deuxième couche (101b) de ladite bande stratifiée absorbante (101) est dépourvue de matériau super-absorbant.

4. Procédé selon l'une quelconque des revendications précédentes, dans lequel ladite deuxième couche (101b) comprend une mousse ou un non-tissé.

5. Procédé selon l'une quelconque des revendications précédentes, dans lequel ladite couche adhésive de contact avec la peau (109) comprend un film polymère muni d'un revêtement adhésif de gel de silicone.

6. Procédé selon l'une quelconque des revendications précédentes, dans lequel ladite couche adhésive de contact avec la peau (109) comprend une pluralité de perforations (110).

7. Procédé selon l'une quelconque des revendications précédentes, dans lequel, dans ladite étape d), ladite pluralité de substrats stratifiés absorbants discrets (107) sont agencés sur ladite bande de couche support (108) à une distance prédéterminée les uns des autres.

8. Procédé selon l'une quelconque des revendications précédentes, dans lequel ladite étape e) d'application d'une couche adhésive de contact avec la peau (109) comprend la disposition d'une bande de couche adhésive de contact avec la peau sur ladite deuxième couche (101b) de chacun desdits substrats stratifiés absorbants discrets (107), ladite bande de couche adhésive de contact avec la peau étant étendue conjointement avec ladite bande de couche support (108).

9. Procédé selon l'une quelconque des revendications précédentes, dans lequel, dans ladite étape f), lesdits pansements individuels sont découpés de telle sorte qu'une partie de bordure (111) formée à partir de ladite couche support et de ladite couche adhésive de contact avec la peau (109) est prévue autour du contour de chacun desdits substrats stratifiés absorbants discrets (107).

10. Procédé selon l'une quelconque des revendications précédentes, ledit procédé étant un procédé automatisé continu.

11. Procédé selon l'une quelconque des revendications précédentes, dans lequel ladite bande stratifiée absorbante (101) a une extension latérale (x) et une extension longitudinale (y) ; ladite extension latérale (x) correspond à la direction machine (MD), et la résistance à la traction de ladite bande stratifiée absorbante (101) est plus élevée dans la direction latérale (x) que dans la direction longitudinale (y).

12. Procédé selon l'u en ne quelconque des revendications précédentes, dans lequel chacune de ladite pluralité de structures de lame de coupe discrètes (106) dudit rouleau de coupe à l'emporte-pièce (104) a une forme choisie parmi une forme en croix, une forme en étoile, une forme en y ou une forme arquée, telle qu'une forme en C.

13. Procédé selon l'une quelconque des revendications précédentes, dans lequel ladite nappe stratifiée absorbante (101) comprend au moins une troisième couche (101c), ladite troisième couche (101c) étant disposée soit au-dessus de ladite première couche (101a), soit entre ladite première couche (101a) et ladite deuxième couche (101b), et ladite troisième couche (101c) étant également pourvue de ladite pluralité d'incisions discrètes (102).

14. Pansement fabriqué selon le procédé selon l'une quelconque des revendications 1 à 13, ledit pansement comprenant une couche de support (108), une couche adhésive de contact avec la peau (109) et un substrat stratifié absorbant (107) comprenant au moins une première couche (107a), une deuxième couche (107b) et une troisième couche (107c) agencée entre lesdites première (107a) et deuxième (107b) couches, ladite première couche (107a) comprenant un matériau super-absorbant et étant pourvue d'une pluralité d'incisions discrètes (102) et ladite deuxième couche (107b) étant disposée en contact avec ladite couche adhésive de contact avec la peau (109) et étant exempte de matériau super-absorbant et d'incisions discrètes (102), ladite troisième couche (107c) étant pourvue d'une pluralité d'incisions discrètes (102).
